(19) Europäisches Patentamt
European Patent Office
Office européen des brevets

(11) EP 2 151 427 B1

(12) **FASCICULE DE BREVET EUROPEEN**

(45) Date de publication et mention de la délivrance du brevet:
**19.12.2012 Bulletin 2012/51**

(51) Int Cl.:
*C07C 231/02* (2006.01) *C07C 231/10* (2006.01)
*C07C 233/18* (2006.01) *C07C 209/48* (2006.01)

(21) Numéro de dépôt: **09290605.6**

(22) Date de dépôt: **04.08.2009**

(54) **Nouveau procédé de synthése de l'agomélatine**

Neues Syntheseverfahren für Agomelatin

New method for synthesising agomelatine

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **05.08.2008 FR 0804463**

(43) Date de publication de la demande:
**10.02.2010 Bulletin 2010/06**

(73) Titulaire: **Les Laboratoires Servier**
**92284 Suresnes Cedex (FR)**

(72) Inventeurs:
- **Bontempelli Pascal**
  **F-76210 Beuzeville-La-Grenier (FR)**
- **Jalenques, Xavier**
  **F-76190 Le Pecq (FR)**
- **Starck, Jérome-Benoit**
  **F-92500 Rueil-Malmaison (FR)**
- **Sery, Jean-Pierre**
  **F-76360 Pissy-Poville (FR)**

(56) Documents cités:
**EP-A- 1 564 202** **FR-A- 1 141 276**
**US-A- 3 062 869**

- **W.H. CAROTHERS ET AL.: "The preparation of some primary amines by the catalytic reduction of nitriles" JOURNAL OF THE AMERICAN CHEMICAL SOCIETY., vol. 47, no. 12, 1925, pages 3051-3057, XP002518637 USAMERICAN CHEMICAL SOCIETY, WASHINGTON, DC. & J. MARCH: "ADVANCED ORGANIC CHEMISTRY" 1985, WILEY & SONS , NEW YORK 198280 * page 815, alinéa 6-28 ***

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 151 427 B1

## Description

La présente invention concerne un nouveau procédé de synthèse industriel de l'agomélatine ou *N*-[2-(7-méthoxy-1-naphtyl)éthyl]acétamide de formule (I) :

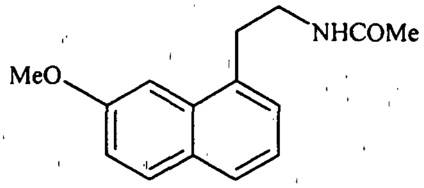

(I)

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-$HT_{2C}$. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Dans le brevet EP 1 564 202, la demanderesse a mis au point une nouvelle voie de synthèse beaucoup plus performante et industrialisable, en seulement quatre étapes, et permettant d'obtenir l'agomélatine de façon très reproductible sous une forme cristalline bien définie.

Le document FR 1 141 276 A décrit la transformation de nitriles hydroaromatiques en amides saturées correspondantes dans un mélange d'anhydride acétique et d'acide acétique ou dans l'anhydride acétique seul.

Le document US 3 062 869 A présente un procédé de réduction de dérivés nitriles en leur composé correspondant monoacétylé, en particulier la réduction du phénylacétonitrile en N-(2-phényléthyl)acétamide dans l'anhydride acétique seul et en présence d'une base.

La demanderesse a maintenant poursuivi ses investigations et mis au point un nouveau procédé de synthèse de l'agomélatine encore plus performant que celui décrit dans l'art antérieur : l'agomélatine est obtenu directement à partir du (7-méthoxy-1-naphtyl) acétonitrile, ce qui permet une synthèse complète réalisable en trois étapes seulement à partir de la 7-méthoxy-1-tétralone. Ce nouveau procédé permet d'obtenir l'agomélatine de façon reproductible et sans nécessiter de purification laborieuse, avec une pureté qui est compatible avec son utilisation comme principe actif pharmaceutique.

Le gain d'une ou plusieurs étapes dans un procédé de synthèse est toujours recherché par l'industriel puisqu'il permet un gain de temps, de rendement et par conséquent un coût final moindre. Cependant, la réduction du nombre d'étapes dans une synthèse n'est pas un exercice trivial surtout lorsque des quantités industrielles sont en jeu : deux étapes rassemblées en une voient le nombre et la quantité de réactifs en présence augmenter, et du fait de la complexification du mélange, la purification du produit de la réaction devient plus difficile. Enfin, la probabilité de voir apparaître des produits secondaires du fait du plus grand nombre de réactifs en présence en même temps est très forte.
La demanderesse a présentement mis au point procédé industriel permettant d'obtenir l'agomélatine directement à partir du (7-méthoxy-1-naphtyl)acétonitrile.

Plus spécifiquement, la présente invention concerne un procédé de synthèse industrielle du composé de formule (I) :

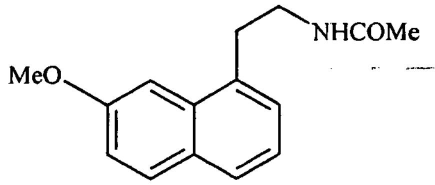

(I)

**caractérisé en ce que** l'on met en réaction le (7-méthoxy-1-naphtyl)acétonitrile de formule (II) :

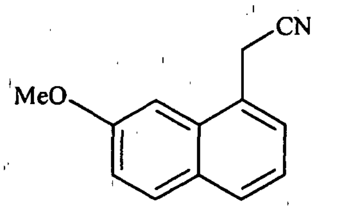

(II)

que l'on soumet à une réduction par l'hydrogène en présence de Nickel de Raney dans un milieu contenant de l'anhydride acétique dans un milieu, réactionnel contenant de l'éthanol et de l'eau, pour conduire au composé de formule (I) que l'on isole sous la forme d'un solide.

Le composé de formule (II) est obtenu par des réactions classiques de la chimie organique. Le composé de formule (II) peut être par exemple obtenu par condensation de l'acide cyanoacétique sur la 7-méthoxy-tétralone puis oxydation du produit de condensation, ainsi que décrit dans les brevets EP1564204 et EP1564205.

De façon préférentielle, la transformation du composé de formule (II) en composé de formule (I) selon l'invention est réalisée sous une pression minimum de 5 bars, et plus préférentiellement réalisée avec une pression comprise entre 10 bars et 50 bars d'hydrogène.

$1 \text{ bar} = 1 \times 10^5 \text{ Pa}$.

Avantageusement la transformation du composé de formule (II) en composé de formule (I) selon l'invention est réalisée entre 25°C et 90°C, et plus particulièrement entre 50°C et 70°C.

La quantité de Nickel de Raney utilisée dans la réaction de transformation du composé de formule (II) en composé de formule (I) est au minimum de 5% massique et plus préférentiellement comprise entre 10% et 20% massique.

Le milieu protique polaire utilisé pour la réaction de transformation du composé de formule (II) en composé de formule (I) contient de l'éthanol et de l'eau. Le milieu réactionnel contient en outre optionnellement de l'acétate de sodium.

Ce procédé est particulièrement intéressant pour les raisons suivantes :

- il permet d'obtenir à l'échelle industrielle le composé de formule (I) en une seule étape à partir du (7-méthoxy-1-naphtyl)acétonitrile avec d'excellents rendements supérieurs à 85%. Ce nouveau procédé permet ainsi l'accès au composé de formule (I) en 3 étapes seulement à partir de la 7-méthoxy-tétralone ;

- le composé de formule (I) obtenu présente de façon reproductible les caractéristiques de la forme cristalline décrite dans le brevet EP 1564202 ;

- les conditions opératoires mises au point permettent de minimiser la formation du produit secondaire majoritaire de la réaction : le *N,N*-bis[2-(7-méthoxy-1 -naphtyl) éthyl]acétamide, qui provient de la dimérisation entre deux intermédiaires réactionnels. Il était en effet a priori très difficile d'envisager l'obtention directe du composé de formule (I) à partir du (7-méthoxy-1-naphtyl)acétonitrile dans des conditions de pureté compatibles avec son utilisation ultérieure pharmaceutique, du fait même de l'existence de cette réaction secondaire dont la proportion augmente de façon dramatique lorsque la réaction est réalisée « one pot ». Des études longues et minutieuses des conditions opératoires ont été nécessaires pour arriver à un taux d'impureté en composé dimérisé acceptable pour une utilisation ultérieure du composé de formule (I) en tant que médicament.

Les exemples ci-dessous illustrent l'invention, mais ne la limitent en aucune façon.

**Example 1 : *N*-[2-(7-Méthoxy-1-naphtyl)éthyl]acétamide**

Dans un réacteur de 670 l sont introduits 85,0 kg de 7-méthoxy-1-tétralone, 60,3 kg d'acide cyanoacétique et 15,6 kg d'acide heptanoïque dans du toluène en présence de 12,7 kg de benzylamine ou 11,0 kg d'aniline. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 90% et une pureté chimique supérieure à 99%.
*<u>Point de fusion</u> : 48-50°C*

**<u>Stade B</u> : (7-Méthoxy-1-naphtyl)acétonitrile**

Dans un réacteur de 670 l sont introduits 12,6 kg de palladium sur charbon à 5% dans du toluène et portés à reflux, puis 96,1 kg de (7-méthoxy-3,4-dihydro-1-naphtalényl) acétonitrile en solution dans du toluène sont ajoutés ainsi que 63,7 kg de méthacrylate d'allyle. La réaction se poursuit à reflux et est suivie par chromatographie en phase vapeur. Lorsque tout le substrat de départ a disparu, le milieu réactionnel est refroidi à l'ambiante puis filtré. Après évaporation du toluène, le résidu solide obtenu est recristallisé dans un mélange éthanol/eau (80/20) pour conduire au produit du titre avec un rendement de 91% et une pureté chimique supérieure à 99%.
*<u>Point de fusion</u> : 83°C*

**<u>Stade C</u> : *N*-[2-(7-Méthoxy-1-naphtyl)éthyl]acétamide**

Dans un réacteur de 8 l sont introduits 136 g de Nickel de Raney, 2,06 l d'éthanol et 0,23 l d'eau. Sous agitation à 70°C et sous 30 bars d'hydrogène, le composé obtenu au Stade B (0,8 kg) en solution dans l'anhydride acétique (2,4 l) est ajouté lentement. A la fin de l'addition, le milieu réactionnel est agité 1 heure sous hydrogène à 30 bars, puis le réacteur est décompressé, et les jus sont filtrés. Après concentration du milieu, on cristallise le résidu dans un mélange éthanol/eau 35/65 pour conduire au produit du titre avec un rendement de 89% et une pureté chimique supérieure à 99%.
*<u>Point de fusion</u> : 108°C*

**<u>Exemple 2</u> : Détermination de la forme cristalline du composé *N*-[2-(7-méthoxy-1-naphtyl)éthyl]acétamide obtenu dans l'Exemple 1**

L'enregistrement des données a été effectué sur le diffractomètre haute résolution D8 de Bruker AXS avec les paramètres suivants : un domaine angulaire 3°-90° en 2θ, un pas de 0,01° et 30 s par pas. La poudre de *N*-[2-(7-méthoxy-1-naphtyl)éthyl]acétamide obtenue dans l'Exemple 1 a été déposée sur un support pour un montage en transmission. La source de rayons X est un tube au cuivre ($\lambda CuK_{\alpha 1}$ = 1,54056 Å). Le montage comporte un monochromateur avant (cristal de Ge(111)) et un détecteur solide résolu en énergie (MXP-D1, Moxtec-SEPH).
Le composé est bien cristallisé : la largeur des raies à mi-hauteur est de l'ordre de 0,07° en 2θ. Les paramètres suivants ont ainsi été déterminés :

- maille cristalline monoclinique,
- paramètres de maille : a = 20,0903 Å, b = 9,3194 Å, c = 15,4796 Å, β = 108,667°
- groupe d'espace : $P2_1/n$
- nombre de molécules dans la maille : 8
- volume de la maille : $V_{maille}$ = 2746,742 $Å^3$
- densité : d = 1,13 $g/cm^3$.

**<u>Exemple 3</u> : Détermination de la forme cristalline du composé *N*-[2-(7-méthoxy-1-naphtyl)éthyl]acétamide obtenu dans l'Exemple 1 par diagramme de diffraction X sur poudre**

La forme cristalline du composé obtenu dans l'Exemple 1 est caractérisée par le diagramme de diffraction X sur poudre suivant, mesuré sur un diffractomètre Siemens D5005 (anticathode de cuivre) et exprimé en termes de distance inter-réticulaire d, d'angle de Bragg 2 thêta, et d'intensité relative (exprimée en pourcentage par rapport à la raie la plus intense) :

| Angle 2 theta (°) | Distance d inter-réticulaire (Å) | Intensité (%) |
|---|---|---|
| 9,26 | 9,544 | 23 |
| 10,50 | 8,419 | 13 |
| 15,34 | 5,771 | 24 |
| 17,15 | 5,165 | 100 |

## Revendications

1. Procédé de synthèse industrielle du composé de formule (I)

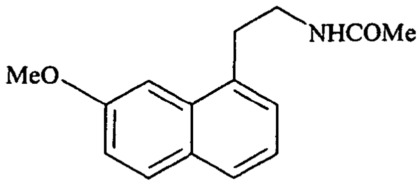

(I)

**caractérisé en ce que** l'on met en réaction le (7-méthoxy-1-naphtyl)acétonitrile de formule (II) :

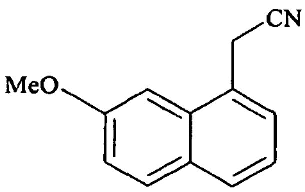

(II)

que l'on soumet à une réduction par l'hydrogène en présence de Nickel de Raney dans un milieu contenant de l'anhydride acétique dans un milieu réactionnel contenant de l'éthanol et de l'eau, pour conduire au composé de formule (I) que l'on isole sous la forme d'un solide.

2. Procédé de synthèse du composé de formule (I) selon la revendication 1 **caractérisé en ce que** la réaction est réalisée sous une pression comprise entre 10 bars et 50 bars d'hydrogène.

3. Procédé de synthèse du composé de formule (I) selon la revendication 1, **caractérisé en ce que** la réaction est réalisée entre 25°C et 90°C.

4. Procédé de synthèse du composé de formule (I) selon la revendication 1, **caractérisé en ce que** la quantité de Nickel de Raney utilisée dans la réaction est comprise entre 10% et 20% massique.

## Claims

1. Process for the industrial synthesis of the compound of formula (I)

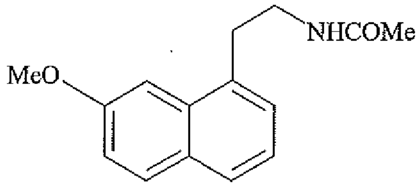

(I),

**characterised in that** there is reacted (7-methoxy-1-naphthyl)acetonitrile of formula (II):

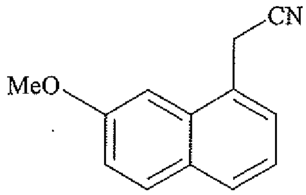

(II),

which is subjected to reduction by hydrogen in the presence of Raney nickel in a medium comprising acetic anhydride in a reaction mixture comprising ethanol and water to yield the compound of formula (I), which is isolated in the form of a solid.

2. Process for the synthesis of the compound of formula (I) according to claim 1, **characterised in that** the reaction is carried out under a pressure of from 10 bars to 50 bars of hydrogen.

3. Process for the synthesis of the compound of formula (I) according to claim 1, **characterised in that** the reaction is carried out at from 25°C to 90°C.

4. Process for the synthesis of the compound of formula (I) according to claim 1, **characterised in that** the amount of Raney nickel used in the reaction is from 10 % to 20 % by weight.

## Patentansprüche

1. Verfahren zur technischen Synthese der Verbindung der Formel (I):

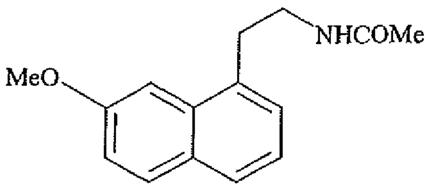

(I)

**dadurch gekennzeichnet, dass** man (7-Methoxy-1-naphthyl)-acetonitril der Formel (II):

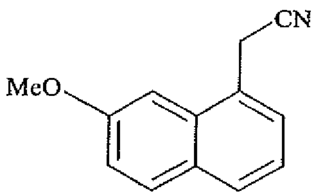

(II)

einer Reduktion mit Wasserstoff in Gegenwart von Raney-Nickel in einem Medium, welches Essigsäureanhydrid in einem Ethanol und Wasser enthaltenden Reaktionsmedium unterwirft zur Bildung der Verbindung der Formel (I), die man in Form eines Feststoffs isoliert.

**2.** Verfahren zur Synthese der Verbindung der Formel (I) nach Anspruch 1, **dadurch gekennzeichnet, dass** die Umsetzung bei einem Wasserstoffdruck zwischen 10 bar und 50 bar durchgeführt wird.

**3.** Verfahren zur Synthese der Verbindung der Formel (I) nach Anspruch 1, **dadurch gekennzeichnet, dass** die Reaktion zwischen 25 °C und 90 °C durchgeführt wird.

**4.** Verfahren zur Synthese der Verbindung der Formel (I) nach Anspruch 1, **dadurch gekennzeichnet, dass** die bei der Reaktion verwendete Menge von Raney-Nickel zwischen 10 Massen-% und 20 Massen-% beträgt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0447285 A
- EP 1564202 A
- FR 1141276 A
- US 3062869 A
- EP 1564204 A
- EP 1564205 A